# EUROPEAN PATENT SPECIFICATION

(11) **EP 3 650 834 B1**
(45) Date of publication and mention of the grant of the patent: **13.03.2024**
(21) Application number: 18204736.5
(22) Date of filing: 06.11.2018
(51) Int. Cl.: G01N 21/17, A61B 5/00, G01N 21/47, G01N 21/49

(54) **APPARATUS, SYSTEM, METHOD, AND COMPUTER PROGRAM FOR ENABLING SPECTROSCOPIC ANALYSIS OF A SAMPLE**
VORRICHTUNG, SYSTEM, VERFAHREN UND COMPUTERPROGRAMM ZUM ERMÖGLICHEN DER SPEKTROSKOPISCHEN ANALYSE EINER PROBE
APPAREIL, SYSTÈME, PROCÉDÉ ET PROGRAMME D'ORDINATEUR POUR PERMETTRE L'ANALYSE SPECTROSCOPIQUE D'UN ÉCHANTILLON

(43) Date of publication of application: 13.05.2020
(73) Proprietor: Nokia Technologies Oy, 02610 Espoo (FI)
(72) Inventor: BITAULD, David, Cambridge, Cambridgeshire CB2 8DL (GB)
(74) Representative: Swindell & Pearson Limited

(56) References cited:
- DE-A1-102015 009 863
- US-A- 5 406 377
- US-A1- 2010 188 496
- US-A1- 2013 102 865
- US-A1- 2018 275 046

## Description

### TECHNOLOGICAL FIELD

Embodiments of the present disclosure relate to an apparatus, system, method, and computer program for enabling spectroscopic analysis of a sample. Some relate to an apparatus, system, method, and computer program for enabling spectroscopic analysis of blood in a blood vessel.

### BACKGROUND

It is useful to obtain information about the molecular composition of samples such as, for example, blood and biological tissue in a subject. It is useful to do this using non-invasive techniques.

DE 10 2015 009863 A1 describes irradiating locally a biological body in a pulsed manner with light from a wavelength range matched to an absorption signature of an analyte. At least part of the light penetrates the body and is absorbed by the analyte. An acoustic wave propagates in the body as a result of the modulated absorption by the analyte. The acoustic wave changes local optical properties of the body. The changes in the optical properties are detected locally by observing a test light radiated into the body. A value of the measured variable of the analyte is deduced from the detected change.

US 2018/275046 A1 describes an excitation beam to generate ultrasonic signals in a sample at an excitation location where a portion of an interrogation beam incident on the sample at the excitation location is returned and is indicative of the generated ultrasonic signals.

US 5 406 377 A describes pulsed electromagnetic radiation impinging on a surface of interest where it is absorbed. The absorption of radiation causes the surface of the sample to expand. This change in dimension is then detected by an interferometer.

### BRIEF SUMMARY

The scope of protection sought for various embodiments of the invention is set out by the independent claims.

The examples and features, if any, described in this specification that do not fall under the scope of the independent claims are to be interpreted as examples useful for understanding various embodiments of the invention.

According to various, but not necessarily all, examples of the present disclosure there is provided an apparatus comprising means for: causing illumination of a sample comprising a structure with first light configured to scatter at one or more interfaces of the structure; causing illumination of the sample with second light configured to excite one or more molecules in the structure to thereby cause thermal expansion of the structure; causing modulation of the second light to thereby modulate thermal expansion of the structure, wherein the second light is caused to be modulated at a frequency of 100 MHz or more; and enabling receipt of first light scattered at the one or more interfaces of the structure during different phases of the modulation of the second light for spectroscopic analysis.

The second light may be caused to be modulated at a frequency whereby pressure waves generated by the modulated thermal expansion of the structure do not propagate to an exterior surface of the structure.

The means may be configured to cause variation of a wavelength of the second light across a wavelength interval.

The second light may have a wavelength which excites one or more of a target molecule.

The following portion of this "Brief Summary" section describes various features that may be features of any of the examples described in the foregoing portion of the "Brief Summary" section. The description of a function should additionally be considered to disclose any means suitable for performing that function.

The means may be configured to cause contemporaneous illumination of the sample with the first light and the second light.

The means may be configured to cause the first and second light to be directed at a common position of the sample.

The means may be configured to cause modulation of an amplitude of the second light to thereby modulate the thermal expansion of the structure.

The means may be configured to: cause illumination of the sample with third light of a fixed wavelength; and to cause modulation of an amplitude of the third light in anti-phase with the modulation of the amplitude of the second light.

The second light may be caused to be modulated at a frequency which is greater than a rate at which the first light is caused to repeat a wavelength sweep across a wavelength interval.

The means may be configured to enable a position of the sample at which the first light and second light are directed to be varied.

The means may be configured to cause receipt of first light scattered at the one or more interfaces of the structure during different phases of the modulation of the second light at least when the first and second light are directed at a first common position and when the first and second light are directed at a second common position, different to the first common position.

In some examples the means may be configured to: determine an amplitude of the modulated thermal expansion of the structure at a plurality of wavelengths within the wavelength interval of the second light; determine an absorption spectrum across the wavelength interval based on the determined amplitude at the plurality of wavelengths; and determine a concentration of at least one target molecule in the structure based on comparison of a known spectrum of the at least one target molecule with the determined absorption spectrum.

In other examples the means may be configured to: determine a first amplitude of the modulated thermal expansion of the structure based on the first light scattered at the one or more interfaces during a period of time when the first and second light are directed at the first common position; determine a first value of absorption of the second light based on the determined first amplitude; determine a second amplitude of the modulated thermal expansion of the structure based on the first light scattered at the one or more interfaces during a period of time when the first and second light are directed at the second common position; determine a second value of absorption of the second light based on the determined second amplitude; determine variation between at least the first and second values of absorption; and determine a concentration of the target molecule in the structure based on the variation between at least the first and second values of absorption.

The means may be configured to cause modulation of the second light at a frequency which enables receipt of first light scattered at the one or more interfaces of the structure during opposing phases of the modulation of the second light.

In some examples the structure may comprise a blood vessel and the one or more molecules may be one or more molecules which can be comprised in blood.

According to various, but not necessarily all, examples of the present disclosure there is provided a system comprising: a first light source configured to illuminate a sample comprising a structure with first light configured to scatter at one or more interfaces of the structure; a second light source configured to illuminate the sample with second light configured to excite one or more molecules in the structure to thereby cause thermal expansion of the structure; a controller configured to control the second light source to modulate the second light to thereby modulate thermal expansion of the structure, wherein the second light is caused to be modulated at a frequency of 100 MHz or more; and a detector configured to receive first light scattered at the one or more interfaces of the structure during different phases of the modulation of the second light for spectroscopic analysis.

In some examples the controller is configured to vary a wavelength of the second light across a wavelength interval.

In some examples the second light has a wavelength which excites one or more of a target molecule in the structure.

In some examples the controller is configured to modulate the second light at a frequency whereby pressure waves generated by the modulated thermal expansion of the structure do not propagate to an exterior surface of the structure.

The system may comprise an interferometer configured to interferometrically compare first light scattered at the one or more interfaces with reference first light.

According to various, but not necessarily all, examples of the present disclosure there is provided a method comprising: causing illumination of a sample comprising a structure with first light configured to scatter at one or more interfaces of the structure; causing illumination of the sample with second light configured to excite one or more molecules in the structure to thereby cause thermal expansion of the structure; causing modulation of the second light to thereby modulate thermal expansion of the structure, wherein the second light is caused to be modulated at a frequency of 100 MHz or more; and enabling receipt of first light scattered at the one or more interfaces of the structure during different phases of the modulation of the second light for spectroscopic analysis.

In some examples the method comprises causing variation of a wavelength of the second light across a wavelength interval.

In some examples the second light has a wavelength which excites one or more of a target molecule in the structure.

In some examples the method comprises causing modulation of the second light at a frequency whereby pressure waves generated by the modulated thermal expansion of the structure do not propagate to an exterior surface of the structure.

According to various, but not necessarily all, examples of the present disclosure there is provided a computer program comprising program instructions stored thereon for performing at least the following: causing illumination of a sample comprising a structure with first light configured to scatter at one or more interfaces of the structure; causing illumination of the sample with second light configured to excite one or more molecules in the structure to thereby cause thermal expansion of the structure; causing modulation of the second light to thereby modulate thermal expansion of the structure, wherein the second light is caused to be modulated at a frequency of 100 MHz or more; and enabling receipt of first light scattered at the one or more interfaces of the structure during different phases of the modulation of the second light for spectroscopic analysis.

In some examples the computer program comprises program instructions stored thereon for performing causing variation of a wavelength of the second light across a wavelength interval.

In some examples the second light has a wavelength which excites one or more of a target molecule in the structure.

In some examples the computer program comprises program instructions stored thereon for performing causing modulation of the second light at a frequency whereby pressure waves generated by the modulated thermal expansion of the structure do not propagate to an exterior surface of the structure.

According to various, but not necessarily all, examples of the present disclosure there is provided an apparatus comprising: at least one processor; and at least one memory including computer program code, the at least one memory and the computer program code configured to, with the at least one processor, cause the apparatus at least to perform: causing illumination of a sample comprising a structure with first light configured to scatter at one or more interfaces of the structure; causing illumination of the sample with second light configured to excite one or more molecules in the structure to thereby cause thermal expansion of the structure; causing modulation of the second light to thereby modulate thermal expansion of the structure, wherein the second light is caused to be modulated at a frequency of 100 MHz or more; and enabling receipt of first light scattered at the one or more interfaces of the structure during different phases of the modulation of the second light for spectroscopic analysis.

In some examples the at least one memory and the computer program code configured to, with the at least one processor, cause the apparatus to perform causing variation of a wavelength of the second light across a wavelength interval.

In some examples the second light has a wavelength which excites one or more of a target molecule in the structure.

In some examples the at least one memory and the computer program code configured to, with the at least one processor, cause the apparatus to perform causing modulation of the second light at a frequency whereby pressure waves generated by the modulated thermal expansion of the structure do not propagate to an exterior surface of the structure.

According to various, but not necessarily all, examples of the present disclosure there is provided a non-transitory computer readable medium comprising program instructions stored thereon for performing at least the following: causing illumination of a sample comprising a structure with first light configured to scatter at one or more interfaces of the structure; causing illumination of the sample with second light configured to excite one or more molecules in the structure to thereby cause thermal expansion of the structure; causing modulation of the second light to thereby modulate thermal expansion of the structure, wherein the second light is caused to be modulated at a frequency of 100 MHz or more; and enabling receipt of first light scattered at the one or more interfaces of the structure during different phases of the modulation of the second light for spectroscopic analysis.

In some examples the non-transitory computer readable medium comprises program instructions stored thereon for performing causing variation of a wavelength of the second light across a wavelength interval.

In some examples the second light has a wavelength which excites one or more of a target molecule in the structure.

In some examples the non-transitory computer readable medium comprises program instructions stored thereon for performing causing modulation of the second light at a frequency whereby pressure waves generated by the modulated thermal expansion of the structure do not propagate to an exterior surface of the structure.

According to various, but not necessarily all, examples of the present disclosure there is provided examples as claimed in the appended claims.

### BRIEF DESCRIPTION

Some example embodiments will now be described with reference to the accompanying drawings in which:
FIG. 1A shows an example of a system;
FIG. 1B shows an example of an interferometer;
FIG. 2 shows an example of a controller;
FIG. 3 shows an example of a method;
FIGS 4A and 4B, 5A and 4B, and 6A and 6B show examples of modulation of light;
FIG. 7 shows an example of a method;
FIG. 8 shows an example of a method; and
FIGS 9A and 9B show examples of the subject-matter described herein in operation.

### DETAILED DESCRIPTION

The FIGS illustrate an apparatus, system, method, and computer program for enabling spectroscopic analysis of a sample to be performed.

The apparatus comprises means for causing illumination of a sample 30, which comprises a structure 32, with first light 13 and with second light 15. The first light 13 is configured to scatter at one or more interfaces 34 of the structure 32. The second light 15 is configured to excite one or more molecules in the structure 32. The one or more molecules may form part of the structure 32 or may be comprised within the structure 32. Exciting the one or more molecules in the structure 32 causes thermal expansion of the structure 32. The means are configured to cause modulation of the second light 15. Modulation of the second light 15 causes modulation of the thermal expansion of the structure 32. The means are configured to enable receipt of the first light 13 which has been scattered at the one or more interfaces 34 of the structure 32 during different phases of the modulation of the second light 15. For example, the means are configured to receive the scattered first light 13 at least at a first time and a second time. The first time corresponds to a time when the modulation of the second light 15 has a first phase and the second time corresponds to a time when the modulation of the second light 15 has a second phase different to the first phase.

Excitation of the one or more molecules, by means of the second light 15, enables a spectroscopic analysis of the sample 30. Performing spectroscopic analysis on first light 13 scattered at the one or more interfaces enables the composition of the sample 30 to be resolved with respect to depth. The use of light, generally, as a means for exciting and detecting enables the depth-resolved spectroscopic analysis to be achieved without direct, physical contact with the sample 30. Non-contact depth-resolved spectroscopy is therefore achieved. Non-contact depth-resolved spectroscopy enables non-invasive measurements of matter which is surrounded by a scattering or absorbing medium. Such non-invasive measurements provide improved convenience.

In some, but not necessarily all, examples a wavelength of the second light 15 is caused to vary across a wavelength interval. In some other, but not necessarily all, examples the second light 15 has a wavelength which excites one or more of a target molecule in the structure 32. For example, the wavelength of the second light 15 may correspond to a spectral line of a target molecule. In still other examples the wavelength of the second light 15 may not be varied across a wavelength interval nor may the second light 15 have a wavelength which is specifically selected in order to excite any particular target molecule.

Resolving a spectrum into its molecular components comprises matching features in the spectrum with distinctive features which are known to identify molecules. Varying the second light 15 across a wavelength interval results in more of such distinctive features being detected, thus making the feature matching a simpler task. Tuning the second light 15 to excite a particular target molecule removes the need for feature matching. Therefore, while varying the second light 15 across a wavelength interval or tuning the second light 15 to excite one or more of a target molecule in the structure 32 are not indispensable for the purpose of achieving non-contact depth-resolved spectroscopy, they do reduce the complexity of, and therefore processing involved in, resolving a spectrum into its molecular components.

In some examples this non-contact depth-resolved spectroscopy has an application in non-invasive blood analysis. In such examples the aforementioned structure 32 comprises a blood vessel and the one or more molecules are one or more molecules which can be comprised in blood. One or the richest sources of information about health is the blood composition. Being able to detect biomarker blood concentration such as, for example, glucose and cortisol or any other suitable biomarker with a high precision and with non-invasive means enables more regular performance of tests and/or continuous monitoring. This enables early detection and ongoing management of chronic conditions such as diabetes. A depth-resolved solution to spectroscopy, as achieved by the examples provided in the present disclosure, is useful because the skin surrounding the blood vessel tends to absorb far more light than the blood itself. This problem is ameliorated by using depth resolution.

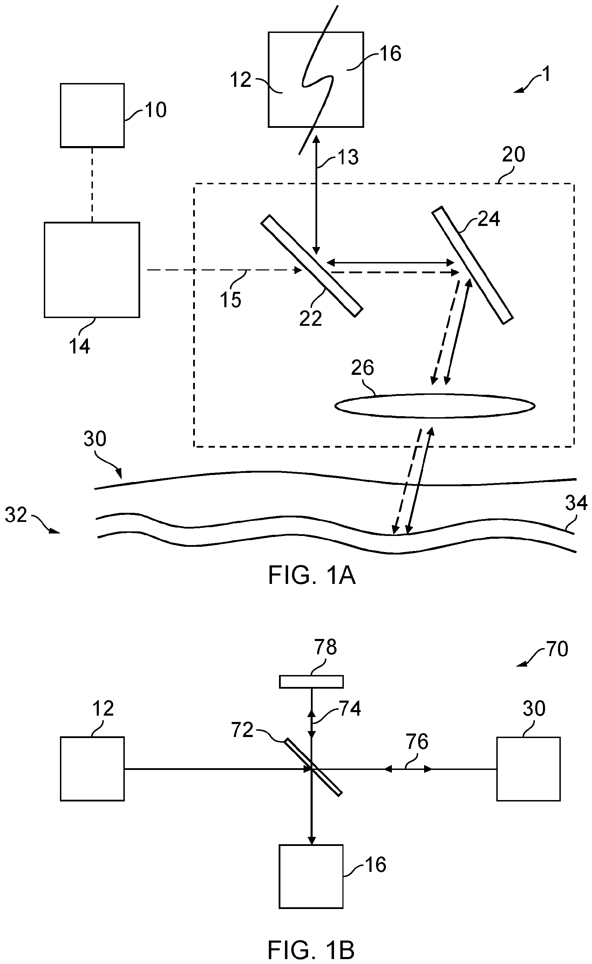

FIG. 1A schematically illustrates an example of a system 1 in accordance with the present disclosure. In some examples the system 1 is comprised in a single device (not shown). The device may be a wearable device.

There is provided a first light source 12 and a second light source 14. The first light source 12 is the source of the first light 13 and the second light source 14 is the source of the second light 15.

In some examples, the first light source 12 is selected to emit light which enables probing of the internal structure 32 of a sample 30. For example, the first light source 12 can be a light source in an optical coherence tomography (OCT) system comprising an interferometer such as, for example, the interferometer 70 shown in FIG. 1B.

FIG. 1B illustrates an example of an interferometer 70 which comprises a reference arm 74 and a sample arm 76. First light 13 may be split by a beam splitter 72 or any other suitable means. The first light 13 is split into two parts. A first part comprising reference first light 13 is directed along the reference arm 74 towards a mirror 78 which directs the reference first light 13 towards a detector 16. A second part is directed along the sample arm 76 towards the sample 30. The first light 13 which is scattered at the sample is directed back along the sample arm 76 and is directed towards the detector 16. The beam splitter 72 recombines the scattered first light 13 and the reference first light 13 to create an interference pattern which can be detected by the detector 16. The interferometer 70 is therefore configured to interferometrically compare the scattered first light 13, having been scattered at the one or more interfaces 34 within the sample 30, with reference first light 13 from the reference arm 74. The reference first light 13 is not scattered at the sample 30. While FIG. 1B illustrates a Michelson interferometer, it is to be appreciated that other double-path interferometers, in which the reference arm 74 and sample arm 76 provide divergent paths for the first light 13, may be used instead.

Returning to FIG. 1A, in some examples the first light source 12 is a tunable source. The first light source 12 may be tunable with respect to the wavelength of the light that it emits. The first light source 12 may be tunable to enable the wavelength of the first light 13 to be varied. An example of a tunable source is a tunable laser. In some, but not necessarily all, examples the first light source 12 is a tunable laser which is selected to enable swept source OCT (SS-OCT). For example, the first light source 12 may be a Fourier-domain mode-locked laser (FDML). In some such examples the first light source 12 performs a wavelength sweep across a wavelength interval which is suitable for scattering at the one or more interfaces 34. The first light source 12 can be selected to be operable in a wavelength band or interval which is not strongly absorbed by human tissues such as between approximately 0.9 and 1.3 micrometers.

Alternatively, the first light source 12 can be a broadband source, which is selected to enable spatially encoded frequency domain OCT (SEFD-OCT) or time domain OCT. The first light source 12 can be selected to be operable in a wavelength band or interval which is not strongly absorbed by human tissues such as between approximately 0.9 and 1.3 micrometers.

In some examples, the second light source 14 is selected for its applicability to molecular spectroscopy. The second light source 14 is therefore selected to emit light in the mid-infrared range which corresponds to the molecular fingerprinting region where each molecule has a complex and distinctive absorption spectrum. In some examples the second light source 14 could be a laser, several lasers for non-linear interactions, or a monochromated light source. In some examples the second light source 14 is a quantum cascade laser.

There is also provided a detector 16 for capturing first light 13 which is scattered at the sample 30. In some examples the detector 16 detects incident light and provides an electrical output or transduces light into an electrical signal. The detector 16 may be, for example, a photodetector. The photodetector may take readings periodically in accordance with a preset or controlled sampling rate. The photodetector may be comprised in an OCT system in conjunction with the first light source 12.

In some examples the system 1 comprises one or more optical elements 20 for directing first and/or second light 13, 15 at the sample 30 and for directing scattered first light 13 to the detector 16. In some examples, one or more optical elements 20 are arranged to cause the first and second light 13, 15, emitted respectively by the first and second light sources 12, 14, to be directed at a common position of the sample 30.

In some examples, by "common position" it is meant that the first and second light 13, 15 illuminate respective areas on the exterior surface of the sample 30 or at an interface 34 of the interior structure 32 which are at least partially, and in some cases substantially wholly, overlapping. In some examples the first and second light 13, 15 are formed as a single beam. In some examples the first and second light 15 illuminate a common position at the exterior surface of the sample 30 and at the interface 34 of the interior structure 32.

The one or more optical elements 20 may comprise one or more amongst a combiner 22, a scanner 24, and a lens 26 as per the example of FIG. 1A. It is to be appreciated that more or less optical elements 20 or their types may be used in systems according to the present disclosure.

The combiner 22 is configured to combine the first light 13 and the second light 15 into a single beam. The single beam enables the first and second light 13, 15 to be directed at, and thus illuminate, one or more common positions on the sample 30. The example of FIG. 1A illustrates a dichroic beam splitter in use as the combiner 22. The dichroic beam splitter may be replaced by a pellicle mirror, a diffraction grating, a prism, etc. or any other suitable means for combing the first and second light 13, 15 into a single beam.

The scanner 24 is configured to scan the first light 13, second light 15, or the single beam of first and second light 13, 15 across the sample 30. The scanner 24 may be an optical scanner such as, for example, a movable reflector. The movable reflector may be a movable mirror. The movable mirror may, for example, be a micro-electromechanical (MEM) element such as, for example, a microscanner. The scanner 24 directs the first light 13, second light 15, or the single beam of first and second light 13, 15 along a direction. Scanning involves moving the reflector to change this direction. In this way the scanner 24 is configured to enable a position of the sample 30 at which the single beam comprising the first and second light 13, 15 is directed to be varied. The reflector may be replaced by any suitable means for varying the position on the sample at which the single beam is directed. For example, the reflector may be replaced by movable refractive optics, acousto-optic deflectors, or electro-optic deflectors. In some examples the scanner 24 could also be removed and means, such as actuators, provided for varying the position of the first and second light sources 12, 14 relative to the sample 30.

The lens 26 focusses the single beam onto a position on the sample 30. The lens 26 also collects light back-scattered from the surface of and one or more interfaces 34 within the sample 30. The lens 26 directs the scattered light towards the detector 16, either directly or via other optical elements 20.

In the example of the system 1 illustrated in FIG. 1A, there is also is provided a controller 10 for controlling the second light source 14.

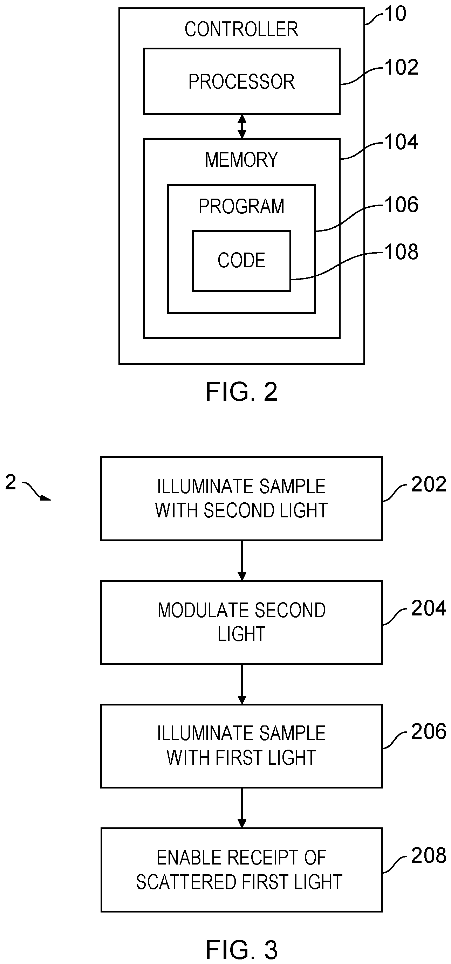

FIG. 2 illustrates an example of the controller 10. Implementation of the controller 10 may be as controller circuitry. The controller 10 may be implemented in hardware alone, have certain aspects in software including firmware alone or can be a combination of hardware and software (including firmware).

As illustrated in FIG. 2 the controller 10 may be implemented using instructions that enable hardware functionality, for example, by using executable instructions of a computer program 106 in a general-purpose or special-purpose processor 102 that may be stored on a computer readable storage medium (disk, memory, etc.) to be executed by such a processor 102.

The processor 102 is configured to read from and write to the memory 104. The processor 102 may also comprise an output interface via which data and/or commands are output by the processor 102 and an input interface via which data and/or commands are input to the processor 102.

The memory 104 stores a computer program 106 comprising computer program instructions (computer program code) 108 that controls the operation of the second light source 14 when loaded into the processor 102. The computer program instructions 108, of the computer program 106, provide the logic and routines that enables the second light source 14 to perform at least: causing illumination of the sample with second light 15 configured to excite one or more molecules in the structure 32 comprised in the sample 30; causing modulation of the second light 15; and, in some examples, causing variation of a wavelength of the second light 15 across a wavelength interval. The processor 102 by reading the memory 104 is able to load and execute the computer program 106.

The system 1 therefore comprises at least one processor 102 and at least one memory 104 including computer program code 108. The at least one memory 104 and the computer program code 108 configured to, with the at least one processor 102, cause the second light source 14 at least to perform: causing illumination of the sample 30 with second light 15 configured to excite one or more molecules in the structure 32 comprised in the sample 30; and causing modulation of the second light 15. In some examples the at least one memory 104 and the computer program code configured to, with the at least one processor 102, cause the second light source 14 to additionally perform causing variation of a wavelength of the second light 15 across a wavelength interval.

The computer program 106 may arrive at the controller 10 via any suitable delivery mechanism. The delivery mechanism may be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid state memory, an article of manufacture that comprises or tangibly embodies the computer program 106. The delivery mechanism may be a signal configured to reliably transfer the computer program 106. The controller 10 may propagate or transmit the computer program 106 as a computer data signal.

In at least some examples, the computer program instructions 108 are for causing the second light source 14 to perform at least the following or for performing at least the following: causing illumination of the sample 30 with second light 15 configured to excite one or more molecules in the structure 32 comprised in the sample 30; causing modulation of the second light 15; and, in some examples, causing variation of a wavelength of the second light 15 across a wavelength interval.

The computer program instructions 108 may be comprised in a computer program, a non-transitory computer readable medium, a computer program product, a machine readable medium. In some but not necessarily all examples, the computer program instructions may be distributed over more than one computer program.

Although the memory 104 is illustrated as a single component/circuitry it may be implemented as one or more separate components/circuitry some or all of which may be integrated/removable and/or may provide permanent/semi-permanent/ dynamic/cached storage.

Although the processor 102 is illustrated as a single component/circuitry it may be implemented as one or more separate components/circuitry some or all of which may be integrated/removable. The processor 102 may be a single core or multi-core processor.

In some examples, the first light source 12, second light source 14, and detector 16 are controlled, at least in part, by a common controller. For example, while each may have associated control circuitry, a central controller at least enables coordination between the components of the system 1 so as to enable the system 1 to perform at least the method 2 illustrated in, and described in relation to, FIG. 3. In some examples the controller 10 as shown in FIG. 1A and FIG. 2 is configured to enable the system 1 to perform at least the method 2 illustrated in, and described in relation to, FIG. 3.

In such examples the hereinbefore described computer program instructions 108, of the computer program 106, provide the logic and routines that enables the system to perform at least the method 2 illustrated in, and described in relation to, FIG. 3. The processor 102 by reading the memory 104 is able to load and execute the computer program 106.

The at least one memory 104 and the computer program code configured to, with the at least one processor 102, perform: causing illumination of a sample 30 comprising a structure with first light 13 configured to scatter at one or more interfaces 34 of the structure 32; causing illumination of the sample 30 with second light 15 configured to excite one or more molecules in the structure 32 to thereby cause thermal expansion of the structure 32; causing modulation of the second light 15 to thereby modulate thermal expansion of the structure 32; and enabling receipt of first light 13 scattered at the one or more interfaces 34 of the structure 32 during different phases of the modulation of the second light 15. In some examples the at least one memory 104 and the computer program code configured to, with the at least one processor 102, additionally perform: causing variation of a wavelength of the second light 15 across a wavelength interval.

In at least some examples, the computer program instructions are for performing at least the following: causing illumination of a sample 30 comprising a structure 32 with first light 13 configured to scatter at one or more interfaces 34 of the structure 32; causing illumination of the sample 30 with second light 15 configured to excite one or more molecules in the structure 32 to thereby cause thermal expansion of the structure 32; causing modulation of the second light 15 to thereby modulate thermal expansion of the structure 32; and enabling receipt of first light 13 scattered at the one or more interfaces 34 of the structure 32 during different phases of the modulation of the second light 15.

In some examples the computer program instructions are configured to perform causing variation of a wavelength of the second light 15 across a wavelength interval.

In some examples a wavelength sweep across the wavelength interval in which the first light source 12 is operable is caused by the controller 10. The controller 10 can control the rate of the sweep and the repetition rate of the sweep.

In some examples a sampling rate of the detector 16 is controlled by the controller 10.

In some examples the detector 16 is controlled by the controller 10 to receive first light 13 scattered at the one or more interfaces 34 of the structure 32 during different phases of modulation of the second light 15 whilst the first and second light 13, 15 are directed at a first common position and also while the first and second light 13, 15 are directed at a second common position.

In some examples controller controls the optical elements too. For example, the scanner can be controlled by the controller to cause the first and second light 13, 15 to be sequentially directed at a series of positions, the first and second light 13, 15 being at each time directed to the same position as one another.

References to "computer-readable storage medium", "computer program product", "tangibly embodied computer program", etc. or a "controller", "computer", "processor", etc. should be understood to encompass not only computers having different architectures such as single/multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term "circuitry" may refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions and
(c) hardware circuit(s) and or processor(s), such as a microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

FIG. 3 schematically illustrates an example of a method 2 of enabling spectroscopic analysis to be performed in accordance with the present disclosure. In some examples this method 2 allows for depth resolution in the spectroscopic analysis. The spectroscopic analysis is performed on a sample comprising a structure such as the sample 30 illustrated in FIG. 1A. The method 2 may be performed by the controller 10.

The method 2 comprises causing illumination of the sample 30 with second light 15 configured to excite one or more molecules in the structure 32 to thereby cause thermal expansion of the structure 32 as per block 202.

The second light 15 is absorbed by the one or more molecules and thus the molecules gain kinetic energy. As a result of the increase in kinetic energy, the amplitude of vibration of the one or more molecules increases and thus the average separation between molecules increases. As a result, the structure 32, in which the molecules are comprised, expands. Where the structure 32 is, for example, a blood vessel, the excitation of molecules within the blood, such as glucose and cortisol or any other molecules, causes the blood vessel to dilate.

The second light 15 may be absorbed differently at different points within the sample 30. The absorption at different points within the sample 30 is dependent on the molecular composition of the sample 30 at those points. The absorption depends on the respective absorbances of the one or more molecules at the wavelength of the second light 15 and the respective concentrations of the one or more molecules.

The method 2 comprises causing modulation of the second light 15 to thereby modulate thermal expansion of the structure 32 as per block 204.

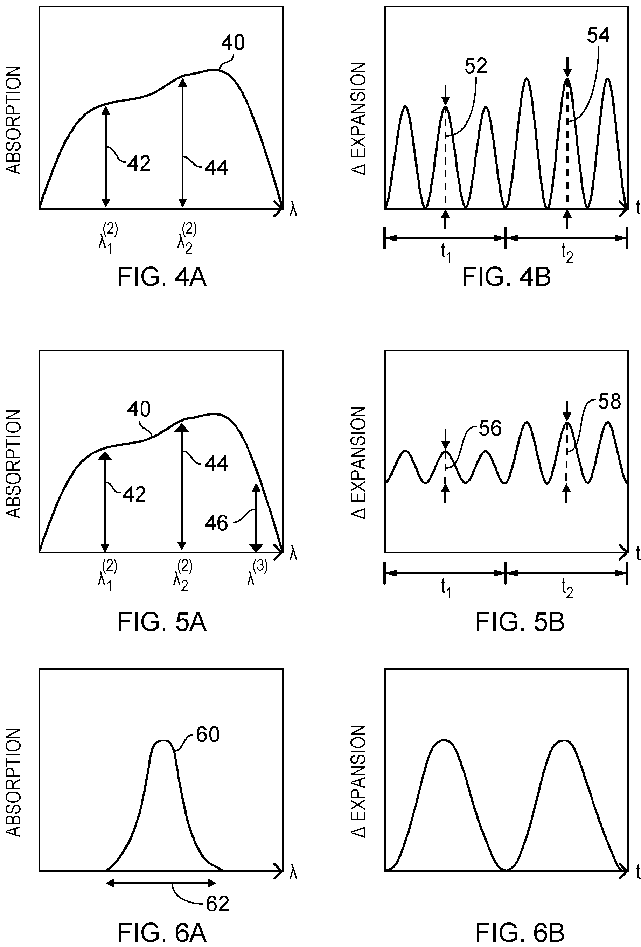

A reduction in the excitation of the one or molecules causes the amount of thermal expansion to decrease. For example, while molecules in blood in a blood vessel are excited, the blood vessel is dilated and as the excitement of the molecules in blood in the blood vessel is reduced, the dilated blood vessel begins to contract. Modulation of the second light 15 comprises increasing and decreasing the excitation of the one or more molecules. Therefore, modulation of the second light 15 causes the thermal expansion of the structure 32 to increase and decrease accordingly. The position of an interface 34 of the structure 32 varies with the thermal expansion of the structure 32. The method 2 involves modulating the second light 15 in order to modulate the thermal expansion in a manner which causes vibration of one or more interfaces 34 of the structure 32. Different approaches to modulation of the second light 15 are described below in reference to FIGS 4A, 4B, 5A, 5B, 6A, and 6B.

The method 2 comprises causing illumination of the sample 30 with first light 13 configured to scatter at the one or more interfaces 34 of the structure 32 as per block 206.

In some examples the contemporaneous illumination of the sample 30 with first and second light 13, 15 is caused. The illumination of the sample 30 with the first light 13 may be sustained whilst the second light 15 is modulated. Illumination of the sample 30 with first light 13 configured to scatter at one or more interfaces 34 of the structure 32 enables determination of the thermal expansion of the structure 32 during different phases of the modulation of the second light 15.

The method 2 comprises enabling receipt of first light 13 scattered at the one or more interfaces 34 of the structure 32 during different phases of the modulation of the second light 15, as per block 208.

In some examples the controller 10 controls the detector 16 to take readings of incident light at times corresponding to different phase of modulation of the second light 15. This may be in accordance with a sampling rate of the detector 16 which is set by the controller 10. The incident light may comprise first light 13 which has been scattered by the one or more interfaces 34. In some examples this first light 13 which is incident on the detector 16 is interferometrically combined with reference first light 13 in order to create an interference pattern. The interference pattern enables a depth map of the concentration of molecules within the sample 30 to be obtained.

In some examples, though not shown in FIG. 3, the method 2 may additionally comprise adjusting a sampling rate of the detector 16 or the modulation frequency of the second light source 14 in order that the scattered first light 13 is received at times which correspond to opposing phases of the modulation of the second light 15. In some examples, said opposing phases correspond to maximum and minimum thermal expansion of the structure 32. For example, the sampling time and rate of the detector 16 may be adjusted to synchronize with the modulation of the second light 15.

In some examples the wavelength or wavelength interval of the second light 15 is selected to excite one or more target molecules.

In some, but not necessarily all, examples the second light source 14 is tunable so that the wavelength of the second light 15 can be varied. The wavelength of the second light 15 can be varied over a wavelength interval. In some examples the method 2 comprises causing variation of the wavelength of second light 15 over the wavelength interval. The wavelength of the second light 15 can be varied over a fixed wavelength interval which excites one or more target molecules. For example, the wavelength interval may be one which contains wavelengths corresponding to one or more spectral lines of the target molecule. In other examples the wavelength interval may be selected to cover distinctive spectral lines of a plurality of molecules. In accordance with this example the hereinbefore described method results in obtaining a depth scan (A-scan in OCT) of the sample 30 where points in space are correlated with a spectrum over the wavelength interval.

In some, but not necessarily all, other examples the second light source 14 is not tunable with respect to wavelength so that the wavelength of the second light 15 is fixed. The second light source 14 is therefore selected to be one which emits light at the desired wavelength. In accordance with this example the hereinbefore described method results in obtaining a depth scan of the sample 30 where points in space are correlated with values of absorption at the fixed wavelength.

By repeating the depth scan at a plurality of adjacent positions and laterally combining these, a cross-sectional tomograph (B-scan in OCT) of the sample 30 can be achieved. The depth scan can be repeated at a plurality of adjacent positions by causing the first and second light 13, 15 to be sequentially directed at a series of positions. Successive positions of the series of positions can be adjacent each other. These successive positions may be contiguous or partially overlap.

FIGS 4A, 4B, 5A, 5B, 6A, and 6B illustrate examples of the manner in which the second light 15 can be modulated.

FIGS 4A and 4B illustrate an example of amplitude modulation of the second light 15. FIG. 4A shows an example of an absorption spectrum 40 of an arbitrary example molecule. FIG. 4B is a graphical representation of modulation of the thermal expansion of a structure in which the example molecule is comprised.

In FIG. 4A an example is shown in which the amplitude of the second light 15 is modulated at a first wavelength λ⁽²⁾₁ during a first time period t₁ and at a second wavelength λ⁽²⁾₂ during a second, subsequent, time period t₂. The modulations 42, 44 during the first and second periods of time t₁, t₂ are represented by double headed arrows in FIG. 4A. By decreasing the amplitude of the second light 15 illuminating the sample, there is less absorption. Correspondingly, increasing the amplitude of the second light 15 increases the absorption. In the example of FIG. 4A, the example molecule has a greater absorbance at the second wavelength λ⁽²⁾₂ than at the first wavelength λ⁽²⁾₁.

In FIG. 4B the thermal expansion of the structure is shown with respect to time. In this example the modulation of the amplitude is sinusoidal, though it is to be appreciated that the amplitude modulation may be pulsed. Since the example molecule has a greater absorbance at the second wavelength λ⁽²⁾₂, the modulated thermal expansion has a greater amplitude 54 during the second time period t₂ when the example molecule is illuminated with light of the second wavelength λ⁽²⁾₂ than the amplitude 52 during the first time period t₁ when the molecule is illuminated with light of the first wavelength λ⁽²⁾₁.

While the amplitude modulation is shown at two different wavelengths in the example of FIGS 4A and 4B it is to be appreciated that in some examples the amplitude modulation is performed at only one wavelength or is performed at more than two wavelengths.

In examples where the amplitude is modulated at only one wavelength, that wavelength is chosen because it is known that absorbance at that wavelength enables a target molecule to be distinguished from other molecules or at least other molecules which may reasonably be expected to be present within the sample or structure.

In other examples the amplitude modulation may occur at a plurality of wavelengths as the wavelength of the second light 15 is varied across a wavelength interval. At a first wavelength in the interval the amplitude of the second light 15 is modulated to at least a maximum and a minimum. This is repeated at successive wavelengths in the interval.

FIGS 5A and 5B illustrate an example of differential amplitude modulation of the second light 15. FIG. 5A shows an example of an absorption spectrum 40 of an arbitrary example molecule. FIG. 5B is a graphical representation of modulation of the thermal expansion of a structure in which the example molecule is comprised.

FIG. 5A is similar to FIG. 4A however, in this example, the sample is additionally illuminated with a third light of a fixed wavelength λ⁽³⁾. The second light 15 and the third light are both modulated with respect to their amplitudes. The modulation 46 of the amplitude of the third light is in antiphase with the modulation 42, 44 of the amplitude of the second light 15.

The third light, in effect, filters out the contribution of any molecules which have a substantially flat spectra in the wavelength interval over which the second light 15 is varied. A molecule having such a substantially flat spectrum is difficult to identify using second light 15 varied over this wavelength interval. The presence of such molecules in the sample merely disguises the presence of other molecules that can be identified by their spectra in this wavelength interval. By filtering out the contribution of any molecules which have a substantially flat spectra in this wavelength interval, the change in the amplitude of the modulated thermal expansion across the wavelength interval as compared to the amplitude 56, 58 of the modulated thermal expansion becomes more significant and thus less susceptible to being disguised by noise. This is observable by comparing FIG. 5B with FIG. 4B. It is the change in the amplitude across the wavelength interval which provides the spectral profile against which the spectra of known molecules can be compared in order to determine the composition of the sample. Use of differential amplitude modulation therefore has the effect of improving the accuracy by which molecules in the sample can be identified.

Similar to the amplitude modulation illustrated in FIGS 4A and 4B, differential amplitude modulation can alternatively be performed at only one wavelength of the second light 15. The second light 15 and third light may have fixed wavelengths and their amplitudes may be modulated in antiphase.

FIGS 6A and 6B illustrate an example of wavelength (frequency) modulation. The approach of applying a modulation 62 to the wavelength of the second light 15 approach works well when seeking to determine the presence of a target molecule that is distinguished, spectroscopically, by a narrow spectral line width in the absorption spectrum 60 of the target molecule, as shown in FIG. 6A. Molecular collisions broaden spectral line widths. Molecular collisions occur often in liquids or biological tissues. While this modulation approach may therefore work best for spectroscopic analysis of gases it is nevertheless possible to use this modulation approach for analysis of liquids or biological tissues.

In some examples, regardless of the manner of modulation, the second light 15 is modulated at a frequency whereby pressure waves which are generated by the modulated thermal expansion of the structure 32 do not propagate to an exterior surface of the sample 30. Because the frequency of the generated pressure waves is related to the modulation frequency, increasing the modulation frequency causes an increase in the frequency of the pressure waves. Higher frequency pressure waves are more highly attenuated. Therefore, modulating the second light 15 at a higher frequency causes a decrease in the propagation, within the sample 30, of the pressure waves generated by the modulated thermal expansion. The modulation frequency is selected to restrict propagation of the generated pressure waves within the sample 30. The modulation frequency is selected so that the generated pressure waves are highly attenuated by the sample 30.

It is to be understood that the generated pressure waves may propagate to an exterior surface of the sample 30 but their power at this surface will be very low and the amplitude of vibration of the exterior surface that is induced by these pressure waves may not be distinguishable from noise. In some examples the vibration of the exterior surface which is induced by the low-power pressure waves does not facilitate detection or at least does not facilitate reliable detection. For example, the detector 16 may be sensitive enough to detect the amplitude of modulated thermal expansion of the structure 32 but not sensitive enough to detect the amplitude of vibration of an exterior surface of the sample 30 induced by the highly-attenuated, low-power pressure waves which have propagated to the surface from within the sample 30. The amplitude of modulated thermal expansion of the structure 32 is far greater than the amplitude of vibration of the exterior surface of the sample 30.

In some examples the second light 15 is modulated at a frequency which, by means of modulated thermal expansion of the structure 32, induces pressure waves of a type which are highly attenuated so as to restrict propagation of the pressure waves to other interfaces 34 within the sample 30. Because the pressure waves induced by the modulated thermal expansion do not substantially propagate within the sample 30, vibration of the one or more interfaces 34 of the structure 32 remain localized and can therefore be probed directly by the first light 13. This enables depth resolution.

In some examples the second light 15 is modulated at a frequency of 100 MHz or more in order to restrict propagation of induced pressure waves to other interfaces 34 within the sample 30.

In some examples the second light 15 is modulated at a frequency which is greater than the rate at which the first light source 12 repeats a wavelength sweep across a wavelength interval. In some examples, the rate at which the first light source 12 repeats a wavelength sweep across a wavelength interval is 100 kHz.

While high frequency modulation has been described in the foregoing passages, it is to be understood that the method 2 of FIG. 3 and depth-resolved spectroscopy generally can be performed with low frequency modulation of the second light 15.

Low frequency modulation of the second light 15 causes a low frequency modulated thermal expansion. This leads to pressure waves having low frequency and which therefore propagate throughout the sample 30. Measurements therefore cannot be directly localized. A reconstruction algorithm is required to try and invert the propagation to determine the origin of the modulated expansion in order to get a depth resolved spectroscopic measurement.

By modulating the second light 15 at a frequency which induces pressure waves of a frequency which hardly propagates between interfaces 34, a direct spectroscopic measurement can be obtained at the depth where the vibrating interface 34 is found. This results in improved accuracy of the depth resolution and reduces the processing required, therefore lowering processing requirements, because it is not necessary to perform the reconstruction.

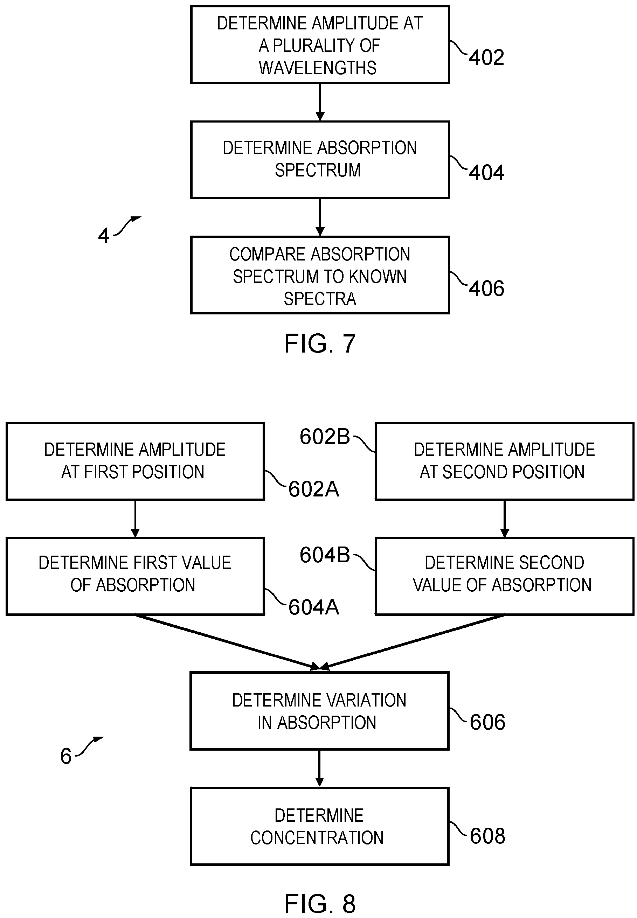

FIGS 7 and 8 schematically illustrate methods 4, 6 for determining a concentration of at least one target molecule in a structure 32. Both methods involve determining an amplitude of the modulated thermal expansion of the structure 32 and determining a concentration of the at least one target molecule in the structure 32 based, at least in part, on the determined amplitude. The determination of the amplitude is repeated for different wavelengths of the second light 15 in FIG.7 and the determination of amplitude is repeated for different common positions of illumination via the first and second light 13, 15. The methods of FIG. 7 and 8 are described in more detail below.

In some examples the methods of FIG. 7 and/or FIG. 8 are performed by the controller 10.

Alternatively, in some examples the methods of FIG. 7 and/or FIG. 8 are performed remotely using data received from the controller 10 or directly from the detector 16. The data may, for example, be measurement data from receiving, at the detector 16, the first light 13 scattered at the one or more interfaces 34 or may, for example, be data produced by the processing of such measurement data at, for example, the controller 10. The measurement data may be an electrical signal resulting from transducing light received by the detector 16.

In some, but not necessarily all, examples the controller 10 is configured to communicate data from the controller 10 with or without local storage of the data in the memory 104 at the controller 10 and with or without local processing of the data by circuitry or processors 102 at the controller 10.

The data may be stored in processed or unprocessed format remotely at one or more devices. The data may be stored in the Cloud.

The data may be processed remotely at one or more devices. The data may be partially processed locally and partially processed remotely at one or more devices.

The data may be communicated to the remote devices wirelessly via short range radio communications such as Wi-Fi or Bluetooth, for example, or over long range cellular radio links. The controller 10 may be coupled to a communications interface such as, for example, a radio transceiver for communication of data.

The controller 10 may be part of the Internet of Things forming part of a larger, distributed network.

The processing of the data, whether local or remote, may be for the purpose of health monitoring, data aggregation, patient monitoring, vital signs monitoring or other purposes.

The processing of the data, whether local or remote, may involve artificial intelligence or machine learning algorithms. The data may, for example, be used as learning input to train a machine learning network or may be used as a query input to a machine learning network, which provides a response. The machine learning network may for example use linear regression, logistic regression, vector support machines or an acyclic machine learning network such as a single or multi hidden layer neural network.

The processing of the data, whether local or remote, may produce an output. The output may be communicated to the controller 10 which may cause an output to be made which is sensible to the subject such as an audio output, visual output or haptic output.

In the example of FIG. 7 the amplitude of modulated thermal expansion of the structure 32 at a plurality of wavelengths within the wavelength interval of the second light 15 is determined as per block 402.

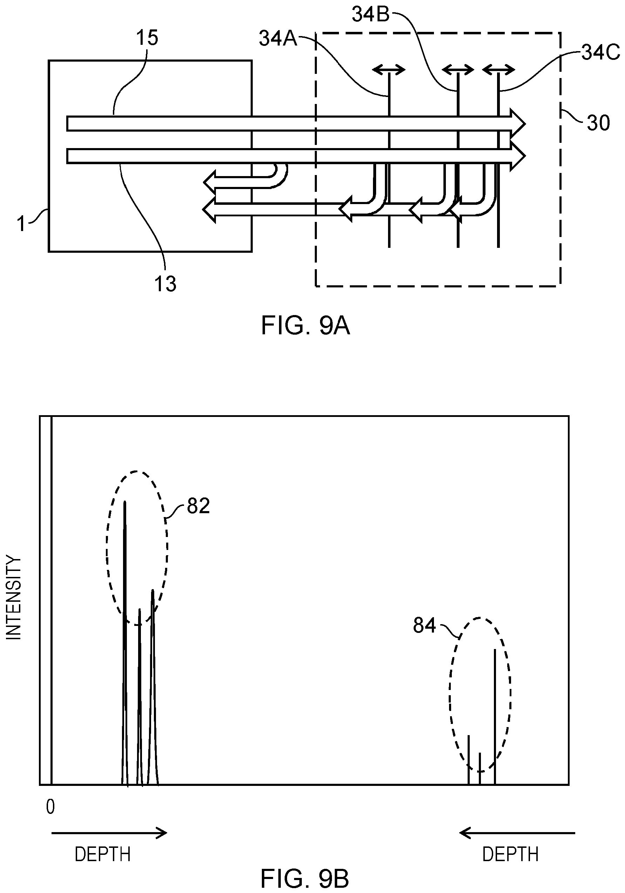

One example of a method of determining this amplitude is described further in relation to FIGS 9A and 9B below and comprises comparing the intensity of principle peaks and sideband peaks in a depth scan. However, it is to be appreciated that it is possible to determine the amplitude of the modulated thermal expansion in other ways. For example, an electrical signal resulting from transducing light received by the detector 16 may be demodulated so that the phase shift resulting from the vibration of the one or more interfaces 34 can be determined. The phase shift is characteristic of the motion of the one or more interfaces 34 of the structure 32 and thus the amplitude of the modulated thermal expansion of the structure 32 can be determined therefrom.

An absorption spectrum across the wavelength interval is determined based on the determined amplitude at the plurality of wavelengths as per block 404.

A spectral profile enables comparison between different absorption spectra in order to determine a composition of the sample 30. In some examples it is therefore the profile of the absorption spectrum which matters rather than the exact values of absorption at each wavelength. In such example it may not be necessary to convert the determined amplitudes into values of absorption at the plurality of wavelengths within the wavelength interval of the second light 15. In other examples the method 4 may additionally comprise, before determining the absorption spectrum, determining a value of absorption at the plurality of wavelengths within the wavelength interval of the second light 15 based on the determined amplitude at the plurality of wavelengths. The absorption spectrum is subsequently determined across the wavelength interval based on the determined values of absorption at the plurality of wavelengths.

The concentration of at least one target molecule in the structure 32 is determined based on the comparison of a known spectrum of at least one target molecule with the determined absorption spectrum as per block 406.

FIG. 8 shows another method 6 of determining the concentration of a target molecule in the structure 32. The method 6 of FIG. 8 may be more applicable when the second light 15 is fixed at a single wavelength, for example a wavelength selected to excite a target molecule.

The method 6 of FIG. 8 comprises determining a first amplitude of the modulated thermal expansion of the structure 32. The first amplitude is determined based on the first light 13 scattered at the one or more interfaces 34 during a period of time when the first and second light 13, 15 are directed at a first common position as per block 602A.

The amplitude may be determined in the same manner as in the method 4 illustrated by FIG. 7.

A first value of the absorption of the second light 15 is determined based on the determined first amplitude as per block 604A.

The method further comprises repeating the aforementioned steps when the first and second light 15 are directed at other common positions as per, for example, blocks 602B and 604B.

The method then comprises determining variation between values of absorption as per block 606 and determining a concentration of the target molecule in the structure 32 based on this variation as per block 608.

The variation of the absorption caused by illumination of different common positions of the sample 30 is assumed to be due to the variation of a concentration of the target molecule. The contribution from other molecules can therefore be filtered out. This is particularly applicable when the other molecules are known to have a reliable and consistent concentration. In some examples, if the variation is within a threshold amount it can be determined that the comparison between absorptions at two points in space is a comparison between absorptions within the structure 32. In such an event the above filtering methodology can be used. In other examples if the variation is outside of a threshold amount it can be determined that the two points in space that are being compared are not within a common structure. In such an event the variation can be disregarded for the purpose of determining the concentration of the target molecule in the structure 32.

The blocks illustrated in the FIGS 3 and 7 or 8 may represent steps in a method and/or sections of code in the computer program 106. The illustration of a particular order to the blocks does not necessarily imply that there is a required or preferred order for the blocks and the order and arrangement of the block may be varied. Furthermore, it may be possible for some blocks to be omitted.

For example, the value of absorption of the second light 15 may be directly proportional to the amplitude of the modulated thermal expansion of the structure 32. In such examples, block 604A and block 604B may be omitted from the method 6. In other examples, vibrations induced by other interfaces or structures within the sample may interfere with thermal expansion of the structure 32. In such examples, a calibrated model may be used to determine the value of absorption of the second light 15 from the amplitude of the modulated thermal expansion of the structure 32 in blocks 604A and 604B.

FIGS 9A and 9B are described in relation to the determination of the amplitude of modulated thermal expansion of the structure 32.

FIG. 9A schematically illustrates the path of first and second light 13, 15. The second light 15 passes through the sample 30. Some of the second light 15 is absorbed by the one or molecules causing, as hereinbefore explained, one or more interfaces 34 within the sample 30 to vibrate. As shown in FIG. 9A, interfaces 34A, 34B, 34C are induced to vibrate. The first light 13 is scattered at each of the interfaces 34A, 34B, 34C. The scattered first light 13 is then interferometrically compared with reference first light 13. The resultant interference pattern is subject of a Fourier transform, as graphically illustrated in FIG. 9B, which enables a depth scan to be obtained.

FIG. 9B graphically represents an example of a Fourier transform of an interference pattern obtained in respect of the illumination, with first and second light 13, 15 in a manner as described in the foregoing, of the sample 30 comprising the interfaces 34A, 34B, 34C. The intensity of the received first light 13 is illustrated against depth. At depths where there is no or little scattering, the intensity of first light 13 is low. At depths where there is significant scattering, such as at the depth of the interfaces 34A, 34B, 34C, the intensity of the first light 13 is relatively high.

On the left side of the graph in FIG. 9B are shown three principle intensity peaks 82 representing the position of the interfaces 34A, 34B, 34C within the sample 30. On the right side of the graph are shown three sideband peaks 84 resulting from the vibration of the interface at which first light 13 is scattered.

In some examples, such as that of FIG. 9B, the sampling rate of the detector 16 and the frequency of modulation of the second light 15 are selected so that the sampling rate of the detector 16 is twice the frequency of modulation of the second light 15 (or a multiple thereof). The sampling rate of the detector 16 may be selected to be greater than the rate at which the first light source 12 repeats a wavelength sweep across a wavelength interval. For example, the sampling rate may enable multiple readings of light incident upon the detector 16 for each wavelength sweep. In some examples more than four readings of the light incident upon the detector 16 can be made for each wavelength sweep. It is to be appreciated that, in many examples, the sampling rate may be much higher than the rate at which the first light source 12 repeats a wavelength sweep across a wavelength interval. For example, 2000 or more readings may be made for each wavelength sweep.

In some examples, receipt of first light 13 which has been scattered from the one or more interfaces 34 occurs contemporaneously with opposing phases of modulation of the second light 15. This causes the greatest separation between the principle peaks 82 and the sideband peaks 84 in the graphical representation of the depth scan, enabling them to be clearly distinguished. The ratio of the intensity of principle peaks 82 to the intensity of the sideband peaks 84 is proportional to the amplitude of the modulated thermal expansion. In some examples the ratio of the intensity of principle peaks 82 to the intensity of the sideband peaks 84 is of the same order as the ratio of the amplitude of the modulated thermal expansion to a center wavelength of the wavelength interval over which the first light 13 is swept. It is therefore possible to determine the amplitude of the modulated thermal expansion from the intensity of the principle and side-band peaks 82, 84. Therefore, ensuring that these peaks 82, 84 can be clearly distinguished from each other improves the confidence and precision with which the amplitude of the modulated thermal expansion can be determined.

In the foregoing, where a structural feature has been described, it may be replaced by means for performing one or more of the functions of the structural feature whether that function or those functions are explicitly or implicitly described.

The above described examples find application as enabling components of personal systems including personal health systems or personal fitness systems and related software and services.

The term "comprise" is used in this document with an inclusive not an exclusive meaning. That is any reference to X comprising Y indicates that X may comprise only one Y or may comprise more than one Y. If it is intended to use "comprise" with an exclusive meaning then it will be made clear in the context by referring to "comprising only one" or by using "consisting".

In this description, reference has been made to various examples. The description of features or functions in relation to an example indicates that those features or functions are present in that example. The use of the term "example" or "for example" or "can" or "may" in the text denotes, whether explicitly stated or not, that such features or functions are present in at least the described example, whether described as an example or not, and that they can be, but are not necessarily, present in some of or all other examples. Thus "example", "for example", "can" or "may" refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

Although embodiments have been described in the preceding paragraphs with reference to various examples, it should be appreciated that modifications to the examples given can be made without departing from the scope of the claims. Features described in the preceding description may be used in combinations other than the combinations explicitly described above.

Although functions have been described with reference to certain features, those functions may be performable by other features whether described or not.

Although features have been described with reference to certain embodiments, those features may also be present in other embodiments whether described or not.

The term "a" or "the" is used in this document with an inclusive not an exclusive meaning. That is any reference to X comprising a/the Y indicates that X may comprise only one Y or may comprise more than one Y unless the context clearly indicates the contrary. If it is intended to use "a" or "the" with an exclusive meaning then it will be made clear in the context. In some circumstances the use of "at least one" or "one or more" may be used to emphasis an inclusive meaning but the absence of these terms should not be taken to infer and exclusive meaning.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon. The invention is only limited by appended claims.

## Claims

1. An apparatus (1) for enabling spectroscopic analysis of a sample comprising means for:
causing illumination (12) of a sample (30) comprising a structure (32) with first light (13)
configured to scatter at one or more interfaces (34) of the structure;
causing illumination (14) of the sample with second light (15) configured to excite one or more molecules in the structure to thereby cause thermal expansion of the structure;
causing modulation of the second light to thereby modulate thermal expansion of the structure, wherein the second light is caused to be modulated at a frequency of 100 MHz or more; and
enabling receipt (16) of first light scattered at the one or more interfaces of the structure
during different phases of the modulation of the second light for spectroscopic analysis.

2. An apparatus as claimed in claim 1 wherein the means are configured to cause variation of a wavelength of the second light across a wavelength interval.

3. An apparatus as claimed in any preceding claim wherein the means are configured to cause contemporaneous illumination of the sample with the first light and the second light and/or wherein the means are configured to cause the first and second light to be directed at a common position of the sample.

4. An apparatus as claimed in any preceding claim wherein the means are configured to cause modulation of an amplitude of the second light to thereby modulate the thermal expansion of the structure.

5. An apparatus as claimed in claim 4 wherein the means are configured to: cause illumination of the sample with third light of a fixed wavelength (λ⁽³⁾); and to cause modulation of an amplitude of the third light in anti-phase with the modulation of the amplitude of the second light.

6. An apparatus as claimed in any preceding claim wherein the means are configured to cause the first light to perform repeated wavelength sweeps across a wavelength interval at a rate of repetition which is less than the frequency at which the the second light is modulated.

7. An apparatus as claimed in any preceding claim wherein the means are configured to enable a position of the sample at which the first light and second light are directed to be varied.

8. An apparatus as claimed in any preceding claim wherein the means are configured to cause receipt of first light scattered at the one or more interfaces of the structure during different phases of the modulation of the second light at least when the first and second light are directed at a first common position and when the first and second light are directed at a second common position, different to the first common position.

9. An apparatus as claimed in any preceding claim wherein the means are configured to:
determine an amplitude (42, 44) of the modulated thermal expansion of the structure at a plurality of wavelengths (λ⁽²⁾1, λ⁽²⁾₂) within the wavelength interval of the second light;
determine an absorption spectrum (40) across the wavelength interval based on the determined amplitude at the plurality of wavelengths; and
determine a concentration of at least one target molecule in the structure based on a comparison of a known spectrum of the at least one target molecule with the determined absorption spectrum.

10. An apparatus as claimed in any preceding claim wherein the means are configured to cause modulation of the second light at a frequency which enables receipt of first light scattered at the one or more interfaces of the structure during opposing phases of the modulation of the second light.

11. An apparatus as claimed in any preceding claim wherein the structure comprises a blood vessel and the one or more molecules are one or more molecules which can be comprised in blood.

12. An apparatus as claimed in any preceding claim wherein:
the means for causing illumination of the sample with first light comprise a first light source (12), the first light source being configured to illuminate the sample comprising the structure with first light configured to scatter at one or more interfaces of the structure;
the means for causing illumination of the sample with second light comprise a second light source (14), the second light source being configured to illuminate the sample with second light configured to excite one or more molecules in the structure to thereby cause thermal expansion of the structure;
the means for causing modulation of the second light comprise a controller (10), the controller being configured to control the second light source to modulate the second light to thereby modulate thermal expansion of the structure, wherein the second light is caused to be modulated at a frequency of 100 MHz or more; and
the means for enabling receipt of scattered first light comprise a detector (16), the detector being configured to receive first light scattered at the one or more interfaces of the structure during different phases of the modulation of the second light for spectroscopic analysis.

13. A method (2) for enabling spectroscopic analysis of a sample comprising:
causing illumination (206) of a sample (30) comprising a structure (32) with first light (13) configured to scatter at one or more interfaces (34) of the structure;
causing illumination (202) of the sample with second light (15) configured to excite one or more molecules in the structure to thereby cause thermal expansion of the structure;
causing modulation (204) of the second light to thereby modulate thermal expansion of the structure, wherein the second light is caused to be modulated at a frequency of 100 MHz or more; and
enabling receipt (208) of first light scattered at the one or more interfaces of the structure during different phases of the modulation of the second light for spectroscopic analysis.

14. An method as claimed in claim 13 wherein the second light has a wavelength tuned to excite one or more of a target molecule.

15. A computer program (106) comprising program instructions (108) stored thereon to cause the apparatus of any of claims 1 to 12 to perform at least the following:
causing illumination (206) of a sample (30) comprising a structure (32) with first light (13) configured to scatter at one or more interfaces (34) of the structure;
causing illumination (202) of the sample with second light (15) configured to excite one or more molecules in the structure to thereby cause thermal expansion of the structure;
causing modulation (204) of the second light to thereby modulate thermal expansion of the structure, wherein the second light is caused to be modulated at a frequency of 100 MHz or more; and
enabling receipt (208) of first light scattered at the one or more interfaces of the structure during different phases of the modulation of the second light for spectroscopic analysis.

## Patentansprüche

1. Vorrichtung (1) zum Ermöglichen einer spektroskopischen Analyse einer Probe, die Mittel für Folgendes umfasst:
Veranlassen einer Beleuchtung (12) einer Probe (30), die eine Struktur (32) umfasst, mit erstem Licht (13), das dazu ausgelegt ist, an einer oder mehreren Schnittstellen (34) der Struktur gestreut zu werden;
Veranlassen einer Beleuchtung (14) der Probe mit zweitem Licht (15), das dazu ausgelegt ist, eine oder mehrere Moleküle in der Struktur zu erregen, um dadurch eine Wärmeausdehnung der Struktur zu veranlassen;
Veranlassen einer Modulation des zweiten Lichts, um dadurch eine Wärmeausdehnung der Struktur zu modulieren, wobei das zweite Licht veranlasst wird, mit einer Frequenz von 100 MHz oder mehr moduliert zu werden; und
Ermöglichen eines Empfangs (16) des ersten Lichts, das an der einen oder den mehreren Schnittstellen der Struktur gestreut wird, während verschiedener Phasen der Modulation des zweiten Lichts für eine spektroskopische Analyse.

2. Vorrichtung nach Anspruch 1, wobei die Mittel dazu ausgelegt sind, eine Variation einer Wellenlänge des zweiten Lichts über ein Wellenlängenintervall zu veranlassen.

3. Vorrichtung nach einem der vorhergehenden Ansprüche, wobei die Mittel dazu ausgelegt sind, eine gleichzeitige Beleuchtung der Probe mit dem ersten Licht und dem zweiten Licht zu veranlassen, und/oder wobei die Mittel dazu ausgelegt sind zu veranlassen, dass das erste und das zweite Licht auf eine gemeinsame Position der Probe gerichtet werden.

4. Vorrichtung nach einem der vorhergehenden Ansprüche, wobei die Mittel dazu ausgelegt sind, eine Modulation einer Amplitude des zweiten Lichts zu veranlassen, um dadurch die Wärmeausdehnung der Struktur zu modulieren.

5. Vorrichtung nach Anspruch 4, wobei die Mittel zu Folgendem ausgelegt sind: Veranlassen einer Beleuchtung der Probe mit drittem Licht einer festen Wellenlänge (λ⁽³⁾); und Veranlassen einer Modulation einer Amplitude des dritten Lichts in Antiphase mit der Modulation der Amplitude des zweiten Lichts.

6. Vorrichtung nach einem der vorhergehenden Ansprüche, wobei die Mittel dazu ausgelegt sind, das erste Licht zu veranlassen, mit einer Wiederholungsrate, die kleiner ist als die Frequenz, mit der das zweite Licht moduliert wird, wiederholte Wellenlängensweeps über ein Wellenlängenintervall durchzuführen.

7. Vorrichtung nach einem der vorhergehenden Ansprüche, wobei die Mittel dazu ausgelegt sind zu veranlassen, dass eine Position der Probe, auf die das erste Licht und das zweite Licht gerichtet werden, variiert wird.

8. Vorrichtung nach einem der vorhergehenden Ansprüche, wobei die Mittel dazu ausgelegt sind, während verschiedener Phasen der Modulation des zweiten Lichts einen Empfang von erstem Licht, das an der einen oder den mehreren Schnittstellen der Struktur gestreut wird, mindestens zu veranlassen, wenn das erste und das zweite Licht auf eine erste gemeinsame Position gerichtet werden und wenn das erste und das zweite Licht auf eine zweite gemeinsame Position gerichtet werden, die sich von der ersten gemeinsamen Position unterscheidet.

9. Vorrichtung nach einem der vorhergehenden Ansprüche, wobei die Mittel zu Folgendem ausgelegt sind:
Bestimmen einer Amplitude (42, 44) der modulierten Wärmeausdehnung der Struktur mit einer Vielzahl von Wellenlängen (λ⁽²⁾₁, λ⁽²⁾₂) innerhalb des Wellenlängenintervalls des zweiten Lichts;
Bestimmen eines Absorptionsspektrums (40) über das Wellenlängenintervall auf Basis der bestimmten Amplitude mit der Vielzahl von Wellenlängen; und
Bestimmen einer Konzentration von mindestens einem Zielmolekül in der Struktur auf Basis eines Vergleichs eines bekannten Spektrums des mindestens einen Zielmoleküls mit dem bestimmten Absorptionsspektrum.

10. Vorrichtung nach einem der vorhergehenden Ansprüche, wobei die Mittel dazu ausgelegt sind, eine Modulation des zweiten Lichts mit einer Frequenz zu veranlassen, die einen Empfang des ersten Lichts, das an der einen oder den mehreren Schnittstellen der Struktur gestreut wird, während entgegengesetzter Phasen der Modulation des zweiten Lichts ermöglicht.

11. Vorrichtung nach einem der vorhergehenden Ansprüche, wobei die Struktur Blutgefäße umfasst und das eine oder die mehreren Moleküle ein oder mehrere Moleküle sind, die in Blut umfasst sein können.

12. Vorrichtung nach einem der vorhergehenden Ansprüche, wobei:
die Mittel zum Veranlassen einer Beleuchtung der Probe mit erstem Licht eine erste Lichtquelle (12) umfassen, wobei die erste Lichtquelle dazu ausgelegt ist, die Probe, die die Struktur umfasst, mit erstem Licht zu beleuchten, das dazu ausgelegt ist, an einer oder mehreren Schnittstellen der Struktur gestreut zu werden;
die Mittel zum Veranlassen einer Beleuchtung der Probe mit zweitem Licht eine zweite Lichtquelle (14) umfassen, wobei die zweite Lichtquelle dazu ausgelegt ist, die Probe mit zweitem Licht zu beleuchten, das dazu ausgelegt ist, ein oder mehrere Moleküle in der Struktur zu erregen, um dadurch eine Wärmeausdehnung der Struktur zu veranlassen;
die Mittel zum Veranlassen einer Modulation des zweiten Lichts eine Steuerung (10) umfassen, wobei die Steuerung dazu ausgelegt ist, die zweite Lichtquelle zum Modulieren des zweiten Lichts zu steuern, um dadurch eine Wärmeausdehnung der Struktur zu modulieren, wobei das zweite Licht veranlasst wird, mit einer Frequenz von 100 MHz oder mehr moduliert zu werden; und
die Mittel zum Ermöglichen eines Empfangs von gestreutem Licht einen Detektor (16) umfassen, wobei der Detektor dazu ausgelegt ist, erstes Licht, das an der einen oder den mehreren Schnittstellen der Struktur gestreut wird, während verschiedener Phasen der Modulation des zweiten Lichts für eine spektroskopische Analyse zu empfangen.

13. Verfahren (2) zum Ermöglichen einer spektroskopischen Analyse einer Probe, das Folgendes umfasst:
Veranlassen einer Beleuchtung (206) einer Probe (30), die eine Struktur (32) umfasst, mit erstem Licht (13), das dazu ausgelegt ist, an einer oder mehreren Schnittstellen (34) der Struktur gestreut zu werden;
Veranlassen einer Beleuchtung (202) der Probe mit zweitem Licht (15), das dazu ausgelegt ist, eine oder mehrere Moleküle in der Struktur zu erregen, um dadurch eine Wärmeausdehnung der Struktur zu veranlassen;
Veranlassen einer Modulation (204) des zweiten Lichts, um dadurch eine Wärmeausdehnung der Struktur zu modulieren, wobei das zweite Licht veranlasst wird, mit einer Frequenz von 100 MHz oder mehr moduliert zu werden; und
Ermöglichen eines Empfangs (208) des ersten Lichts, das an der einen oder den mehreren Schnittstellen der Struktur gestreut wird, während verschiedener Phasen der Modulation des zweiten Lichts für eine spektroskopische Analyse.

14. Verfahren nach Anspruch 13, wobei das zweite Licht eine Wellenlänge aufweist, die zum Erregen von einem oder mehreren eines Zielmoleküls abgestimmt ist.

15. Computerprogramm (106), das darauf gespeicherte Programmanweisungen (108) umfasst, um die Vorrichtung nach einem der Ansprüche 1 bis 12 zu veranlassen, mindestens Folgendes durchzuführen:
Veranlassen einer Beleuchtung (206) einer Probe (30), die eine Struktur (32) umfasst, mit erstem Licht (13), das dazu ausgelegt ist, an einer oder mehreren Schnittstellen (34) der Struktur gestreut zu werden;
Veranlassen einer Beleuchtung (202) der Probe mit zweitem Licht (15), das dazu ausgelegt ist, eine oder mehrere Moleküle in der Struktur zu erregen, um dadurch eine Wärmeausdehnung der Struktur zu veranlassen;
Veranlassen einer Modulation (204) des zweiten Lichts, um dadurch eine Wärmeausdehnung der Struktur zu modulieren, wobei das zweite Licht veranlasst wird, mit einer Frequenz von 100 MHz oder mehr moduliert zu werden; und
Ermöglichen eines Empfangs (208) des ersten Lichts, das an der einen oder den mehreren Schnittstellen der Struktur gestreut wird, während verschiedener Phasen der Modulation des zweiten Lichts für eine spektroskopische Analyse.

## Revendications

1. Appareil (1) permettant de réaliser une analyse spectroscopique d'un échantillon, comprenant des moyens pour :
provoquer l'éclairage (12) d'un échantillon (30) comprenant une structure (32) avec une première lumière (13) configurée pour se diffuser au niveau d'une ou plusieurs interfaces (34) de la structure ;
provoquer l'éclairage (14) de l'échantillon avec une deuxième lumière (15) configurée pour exciter une ou plusieurs molécules dans la structure afin de provoquer une dilatation thermique de la structure ;
provoquer la modulation de la deuxième lumière afin de moduler la dilatation thermique de la structure, dans lequel la deuxième lumière est amenée à être modulée à une fréquence de 100 MHz ou plus ; et
permettre la réception (16) d'une première lumière diffusée au niveau des une ou plusieurs interfaces de la structure pendant différentes phases de la modulation de la deuxième lumière pour analyse spectroscopique.

2. Appareil selon la revendication 1, dans lequel les moyens sont configurés pour provoquer une variation d'une longueur d'onde de la deuxième lumière sur un intervalle de longueur d'onde.

3. Appareil selon l'une des revendications précédentes, dans lequel les moyens sont configurés pour provoquer un éclairage simultané de l'échantillon avec la première lumière et la deuxième lumière, et/ou dans lequel les moyens sont configurés pour faire en sorte que les première et deuxième lumières soient dirigées vers une position commune de l'échantillon.

4. Appareil selon l'une des revendications précédentes, dans lequel les moyens sont configurés pour provoquer la modulation d'une amplitude de la deuxième lumière afin de moduler la dilatation thermique de la structure.

5. Appareil selon la revendication 4, dans lequel les moyens sont configurés :
pour provoquer l'éclairage de l'échantillon avec une troisième lumière d'une longueur d'onde (λ⁽³⁾) fixe ; et
pour provoquer la modulation d'une amplitude du troisième lumière en opposition de phase avec la modulation de l'amplitude de la deuxième lumière.

6. Appareil selon l'une des revendications précédentes, dans lequel les moyens sont configurés pour amener la première lumière à effectuer des balayages de longueur d'onde répétés sur un intervalle de longueur d'onde à un taux de répétition qui est inférieur à la fréquence à laquelle la deuxième lumière est modulée.

7. Appareil selon l'une des revendications précédentes, dans lequel les moyens sont configurés pour permettre de faire varier une position de l'échantillon vers laquelle la première lumière et la deuxième lumière sont dirigées.

8. Appareil selon l'une des revendications précédentes, dans lequel les moyens sont configurés pour provoquer la réception d'une première lumière diffusée au niveau des une ou plusieurs interfaces de la structure pendant différentes phases de la modulation de la deuxième lumière, au moins lorsque les première et deuxième lumières sont dirigées dans une première position commune et lorsque les première et deuxième lumières sont dirigées dans une deuxième position commune différente de la première position commune.

9. Appareil selon l'une des revendications précédentes, dans lequel les moyens sont configurés :
pour déterminer une amplitude (42, 44) de la dilatation thermique modulée de la structure à une pluralité de longueurs d'onde (λ⁽²⁾₁, λ⁽²⁾₂) dans l'intervalle de longueur d'onde de la deuxième lumière ;
pour déterminer un spectre d'absorption (40) sur l'intervalle de longueur d'onde en fonction de l'amplitude déterminée à la pluralité de longueurs d'onde ; et
pour déterminer une concentration d'au moins une molécule cible dans la structure en fonction d'une comparaison d'un spectre connu de l'au moins une molécule cible avec le spectre d'absorption déterminé.

10. Appareil selon l'une des revendications précédentes, dans lequel les moyens sont configurés pour provoquer la modulation de la deuxième lumière à une fréquence qui permet la réception d'une première lumière diffusée au niveau des une ou plusieurs interfaces de la structure pendant des phases opposées de la modulation de la deuxième lumière.

11. Appareil selon l'une des revendications précédentes, dans lequel la structure comprend un vaisseau sanguin, et les une ou plusieurs molécules sont une ou plusieurs molécules pouvant être présentes dans le sang.

12. Appareil selon l'une des revendications précédentes, dans lequel :
les moyens pour provoquer l'éclairage de l'échantillon avec une première lumière comprennent une première source de lumière (12), la première source de lumière étant configurée pour éclairer l'échantillon comprenant la structure avec une première lumière configurée pour se diffuser au niveau d'une ou plusieurs interfaces de la structure ;
les moyens pour provoquer l'éclairage de l'échantillon avec une deuxième lumière comprennent une deuxième source de lumière (14), la deuxième source de lumière étant configurée pour éclairer l'échantillon avec une deuxième lumière configurée pour exciter une ou plusieurs molécules dans la structure afin de provoquer une dilatation thermique de la structure ;
les moyens pour provoquer la modulation de la deuxième lumière comprennent un dispositif de commande (10), le dispositif de commande étant configuré pour commander la deuxième source de lumière pour moduler la deuxième lumière afin de moduler la dilatation thermique de la structure, dans lequel la deuxième lumière est amenée à être modulée à une fréquence de 100 MHz ou plus ; et
les moyens pour permettre la réception d'une première lumière diffusée comprennent un détecteur (16), le détecteur étant configuré pour recevoir une première lumière diffusée au niveau des une ou plusieurs interfaces de la structure pendant différentes phases de la modulation de la deuxième lumière pour analyse spectroscopique.

13. Procédé (2) permettant de réaliser une analyse spectroscopique d'un échantillon comprenant les étapes suivantes :
provoquer l'éclairage (206) d'un échantillon (30) comprenant une structure (32) avec une première lumière (13) configurée pour se diffuser au niveau d'une ou plusieurs interfaces (34) de la structure ;
provoquer l'éclairage (202) de l'échantillon avec une deuxième lumière (15) configurée pour exciter une ou plusieurs molécules dans la structure afin de provoquer une dilatation thermique de la structure ;
provoquer la modulation (204) de la deuxième lumière afin de moduler la dilatation thermique de la structure, dans lequel la deuxième lumière est amenée à être modulée à une fréquence de 100 MHz ou plus ; et
permettre la réception (208) d'une première lumière diffusée au niveau des une ou plusieurs interfaces de la structure pendant différentes phases de la modulation de la deuxième lumière pour analyse spectroscopique.

14. Procédé selon la revendication 13, dans lequel la deuxième lumière a une longueur d'onde réglée pour exciter un ou plusieurs molécules cibles.

15. Programme informatique (106) comprenant des instructions de programme (108) stockées sur celui-ci pour amener l'appareil de l'une des revendications 1 à 12 à effectuer au moins ce qui suit :
provoquer l'éclairage (206) d'un échantillon (30) comprenant une structure (32) avec une première lumière (13) configurée pour se diffuser au niveau d'une ou plusieurs interfaces (34) de la structure ;
provoquer l'éclairage (202) de l'échantillon avec une deuxième lumière (15) configurée pour exciter une ou plusieurs molécules dans la structure afin de provoquer une dilatation thermique de la structure ; provoquer la modulation (204) de la deuxième lumière afin de moduler la dilatation thermique de la structure, dans lequel la deuxième lumière est amenée à être modulée à une fréquence de 100 MHz ou plus ; et
permettre la réception (208) d'une première lumière diffusée au niveau des une ou plusieurs interfaces de la structure pendant différentes phases de la modulation de la deuxième lumière pour analyse spectroscopique.
